# EUROPEAN PATENT SPECIFICATION

(11) **EP 3 173 487 B1**
(45) Date of publication and mention of the grant of the patent: **20.02.2019**
(21) Application number: 15196591.0
(22) Date of filing: 26.11.2015
(51) Int. Cl.: C12Q 1/34

(54) **DEVICE FOR DETECTION OF HALOGENATED HYDROCARBONS**
VORRICHTUNG ZUM NACHWEIS VON HALOGENIERTEN KOHLENWASSERSTOFFEN
DISPOSITIF DE DÉTECTION D'HYDROCARBURES HALOGÉNÉS

(43) Date of publication of application: 31.05.2017
(73) Proprietor: Masarykova univerzita, 601 77 Brno (CZ)
(72) Inventor: Bidmanova, Sarka, 67801 Blansko (CZ); Prokop, Zbynek, 61200 Brno (CZ); Damborsky, Jiri, 62800 Brno (CZ); Dürkop, Axel, 84088 Neufahrn in Niederbayern (DE); Wolfbeis, Otto S., 93051 Regensburg (DE)
(74) Representative: Hartvichova, Katerina

(56) References cited:
- WO-A1-01/36665
- US-A1- 2008 102 501
- US-A1- 2014 235 501
- SARKA BIDMANOVA ET AL: "Development of an enzymatic fiber-optic biosensor for detection of halogenated hydrocarbons", ANALYTICAL AND BIOANALYTICAL CHEMISTRY, SPRINGER, BERLIN, DE, vol. 398, no. 5, 19 August 2010 (2010-08-19), pages 1891-1898, XP019839574, ISSN: 1618-2650
- TANA KOUDELAKOVA ET AL: "Haloalkane dehalogenases: Biotechnological applications", BIOTECHNOLOGY JOURNAL, vol. 8, no. 1, 11 January 2013 (2013-01-11), pages 32-45, XP055249507, DE ISSN: 1860-6768, DOI: 10.1002/biot.201100486

## Description

### Field of Art

The present invention relates to a device for detection of halogenated hydrocarbons based on pH indicator stripes carrying an immobilized enzyme.

### Background Art

Halogenated compounds represent one of the most important groups of industrially produced chemicals. Because of large-scale manufacture and use, they belong to the widespread contaminants found in air, water, soil and sediment. Many of these compounds are halogenated aliphatic hydrocarbons, comprising different haloalkanes, haloalkenes, haloalkynes, haloalcohols, haloamides or haloethers.

The most hazardous halogenated aliphatic hydrocarbons are brominated and chlorinated compounds, containing between one and three carbon atoms, which are released from industrial and agricultural sources. They have been commonly used as herbicides, insecticides, fungicides, solvents in the dry-cleaning operations and lacquer industries, cleaning agents, fumigants, gasoline additives, degreasers and intermediates for chemical syntheses. Also biotic and abiotic processes can be natural sources of halogenated compounds. Bromine-containing hydrocarbons are synthesized abundantly by marine organisms, e.g., by algae, sponges, bryozans and bacteria. Terrestrial organisms, e.g., plants, fungi, lichens, bacteria and insects, mostly produce chlorinated compounds. Iodinated metabolites occur less frequently and biogenic fluorinated compounds are very rare. Thousands of tons of halogenated aliphatic hydrocarbons have been also released during natural abiogenic processes, e.g., volcano eruptions and other geothermal processes, biomass burning, crushing of rocks and minerals in mining operations.

Halogenated aliphatics are prevalent groundwater contaminants and significant components of hazardous wastes and landfill leachates. They are also found in a surface water in typical concentrations ranging from 0.1 µg·l⁻¹ to 6.0 µg·l⁻¹, in a food and in the atmosphere. The concentrations observed in urban air are 0.03 µg·l⁻¹ to 0.1 µg·l⁻¹. Maximum concentrations of these most commonly found contaminants in water supply wells are several to several hundred micrograms per liter.

Halogenated aliphatic hydrocarbons have undesirable negative effects on the human health and the environment. Their toxicity is generally not caused by the compounds themselves, but by metabolic intermediates alkylating proteins or nucleic acids. A wide range of halogenated aliphatics, e.g., 1,2-dichloroethane, 1,2-dibromoethane, 1,2,3-trichloropropane, γ-hexachlorocyclohexane, have been currently classified as potentially mutagenic, carcinogenic and toxic to the liver, kidney or neural system. Acute overexposure to halogenated compounds has serious health consequences for humans, including skin irritation, depression of central nervous system, heart failure, damage of liver and kidneys and increased rates of cancer. Persistence of halogenated hydrocarbons in the environment and tendency to accumulation in living organisms can lead to chronic exposure of humans resulting in higher incidence of tumors and cardiac malformations.

The adverse health effects and increasing production of halogenated compounds result in a demand for control of their levels in the environment. Therefore rapid and accurate *in situ* methods for detecting and measuring concentrations of hazardous halogenated chemicals in air, soil and particularly in water are required to provide early warning systems or can monitor the progress of various remediation treatments.

Conventional analytical methodologies for accurate determination of halogenated hydrocarbons in humans and the environment were developed in the past forty years. Most halogenated hydrocarbons are relatively volatile, non-polar and thermally stable compounds that can be determined by gas chromatography. The whole procedure involves several steps: sampling, transport to a specialized laboratory coupled with the storage of the samples, the sample pretreatment followed by separation and detection of target contaminant. The most time-consuming and labour-intensive task is sample preparation, i.e., isolation and/or pre-concentration of an analyte. The techniques widely applied in air analysis are sorbent and cryogenic sampling, followed by thermal desorption or solvent extraction. Various methods for water samples can be used, e.g., purge and trap analysis, liquid-liquid extraction and solid-phase microextraction. The analysis of soil and sediment requires the use of Soxhlet, solid-phase or supercritical fluid extraction. Subsequently, clean-up is performed with different adsorbents to remove interfering substances from an analyte because the extraction methods are not sufficiently selective. Gas chromatography is applied for separation of individual compounds from the extracts of environmental samples. Electron capture and mass spectrometric detection is employed to identify and quantify the target analyte. Better resolution can be achieved by using time-of-flight or high-resolution mass spectrometry. Bidmanova et al. describe a haloalkane dehalogenase fiber-optic biosensor for the detection of halogenated hydrocarbons (Bidmanova, S., Chaloupkova, R., Damborsky, J., Prokop, Z. (2010): Development of an enzymatic fiber-optic biosensor for detection of halogenated hydrocarbons. Anal Bioanal Chem. 398: 1891-1898).

The aforementioned techniques require sophisticated and expensive equipment, highly trained staff and importantly may require a few days or even a few weeks to obtain the results of these tests. Therefore, there is a growing need for an easy, portable, versatile and quick method for detection and quantification of halogenated hydrocarbons, which does not require highly trained staff and complex equipment.

### Disclosure of the Invention

The present invention introduces a novel device for detection of halogenated aliphatic hydrocarbons. This device is preferably in the form of biosensor test stripes. Hydrolytic conversion of the substrate by the enzyme haloalkane dehalogenase is accompanied by a chemical change such as decrease of pH or increase of halide ions which can be detected using suitable indicators (such as pH indicator or halide ion indicator) contained in the device.

Object of the present invention is a device for detection of halogenated aliphatic hydrocarbons, which contains a paper carrier provided with at least one indicator selected from a pH indicator with a pH-indicating change within the pH range of 4.0 to 10.0, and an indicator of halide ions, and at least one enzyme haloalkane dehalogenase (EC 3.8.1.5) immobilized on said carrier.

The halogenated aliphatic hydrocarbons are preferably selected from C1-C18 alkanes, C2-C18 alkenes, C3-C18 cycloalkanes, wherein the alkane, alkene or cycloalkane is substituted by at least one halogen (selected from F, Cl, Br, I) and optionally further substituted by one or more (e.g., one to five) substituents F, Cl, Br, I, OH, COOH, CN, COO(C1-C4 alkyl), C(O)N(H, C1-C6 alkyl, C6-C10 aryl), N(H, C1-C6 alkyl, C6-C10 aryl)-C(O)-(C1-C4 alkyl), O(C1-C6 alkyl, C2-C6 alkenyl, C1-C6 haloalkyl, C2-C6 haloalkenyl), S(C1-C6 alkyl, C2-C6 alkenyl, C1-C6 haloalkyl, C2-C6 haloalkenyl).

In one embodiment, the halogenated aliphatic hydrocarbons may be selected from the following table (Table 1):

| **Chemical** | **Examples** | **Applications** |
|---|---|---|
| α-haloalkanes | chloromethane, chloroethane, 1-chlorobutane, 1-chlorohexane, bromomethane, bromoethane, 1-bromopropane, 1-bromobutane, 1-bromopentane, 1-bromohexane, 1-bromoheptane, iodomethane, 1-iodoethane, 1-iodopropane, 1-iodobutane, 1-iodohexane | Refrigerants, gasoline additives, chemical intermediates, solvents, propellants, blowing agents, thickening agents, fire extinguishers, local anesthetics, pesticides |
| β-haloalkanes | 2-chloropropane, 2-chlorobutane, 2-bromopropane, 2-bromobutane, 2-bromopentane, 2-bromohexane, 2-bromoheptane, 2-iodobutane | Agents in organic synthesis, solvents |
| Dihaloalkanes | 1,2-dichloroethane, 1,3-dichloropropane, 1,3-dichlorobutane, 1,5-dichloropentane, 1,6-dichlorohexane, 1,9-dichlorononane, 1-bromo-2-chloroethane, 2-bromo-1-chloro-propane, 1,1-dibromomethane, 1,2-dibromo-ethane, 1,2-dibromopropane, 1,3-dibromo-propane, 1,2-dibromobutane, 1,3-dibromobutane, 1,3-diiodopropane | Chemical intermediates, gasoline additives, solvents, agents in organic synthesis, gauge fluids, pesticides |
| Trihaloalkanes | 1,2,3-trichloropropane, 1,2-dibromo-3-chloropropane, 1,2,3-tribromopropane | Solvents, chemical intermediates, cleaning and degreasing agents, paint and varnish removers, pesticides |
| β-methylated haloalkanes | 1-chloro-2-methylpropane, 1-bromo-3-chloro-2-methylpropane, 1-bromo-2-methylpropane | Chemical intermediates |
| Halocycloalkanes | Chlorocyclopentane, chlorocyclohexane, bromocyclohexane, (bromomethyl)cyclohexane | Agents in organic synthesis |
| Haloalkenes | 3-chloropropene, 3-chloro-2-methylpropene, 1,3-dichloropropene, 2,3-dichloropropene | Chemical intermediates, pesticides |
| Haloesters | Methyl 2-bromopropionate, ethyl 2-bromopropionate, ethyl 2-bromobutyrate, methyl 3-bromo-2-methyl propionate, ethyl 3-bromo-2-methyl propionate, methyl 2,4-dibromobutyrate | Agents in organic synthesis |
| Halonitriles | 4-bromobutanenitrile, 4-chlorobutanenitrile | |
| Haloamides | 2-bromo-*N*-phenylpropanamide, ethyl [(2-bromopropanoyl) amino]acetate, *tert*-butyl (2*S*)-2-[(2-bromopropanoyl) amino]propanoate | |
| Haloacids | Fluoroacetic acid, chloroacetic acid, 3-chloropropionic acid, 2-chlorobutyric acid | Chemical intermediates, pesticides |
| Haloalcohols | 3-chloropropan-1-ol, 4-chlorobutan-1-ol, 6-chlorohexan-1-ol, 2-bromoethan-1-ol, 3-bromopropan-1-ol, 6-bromohexan-1-ol | Chemical intermediates |
| Haloepoxides Haloethers | Epichlorohydrin, epibromohydrin 2-chloroethylvinylether, bis(2-chloroethyl) ether | Chemical intermediates Chemical intermediates, solvents, pesticides |
| Haloalkylsulfide | Bis(2-chloroethyl) sulfide | Chemical warfare agent |

In one embodiment, the halogenated hydrocarbon to be detected is selected from the group comprising 1,2-dibromoethane, bis(2-chloroethyl) ether, sulfur mustard (bis(2-chloroethyl) sulfide).

The indicator for the presence of halogenated aliphatic hydrocarbons can be selected from a pH indicator with a pH-indicating change (e.g., absorbance or fluorescence change) within the pH range of 4.0 to 10.0, preferably 6.0 to 8.6, and an indicator of halide ions (chlorides, bromides, iodides or fluorides). Suitable indicators are well known in the art and may include common pH indicators with a colour change, which are incorporated in commercially available pH stripes; further absorbance and fluorescence-based pH indicators such as phenol red, bromothymol blue, alizarin red, 9-amino-6-chloro-2-methoxyacridine, aurin, benzaurin, brilliant yellow, bromocresol purple, bromophenol red, bromoxylenol blue, calcein, calmagite, chrysoidin, *o*-cresolbenzein, *o-*cresolphthalein, *m*-cresol purple, *o*-cresol red, curcumin, 3,6-dihydroxyphthalimide, dixylenolphthalein, ethyl *bis*(2,4-dinitrophenyl)acetate, heptamethoxy red, indophenol, luminol, α-naphtholphthalein, neutral red, nitrazine yellow, *p*-nitrobenzhydrazide, *o*-nitrophenol, Orange II, phenolbenzein, phenolmalein, phenolphthalein, pinachrome, quinoline blue, rhodol green, *p*-rosolic acid, rubrophen, rhodol green, salicylaldehyde semicarbazone, solochrome violet RS, tetrabromophenolphthalein, thymolbenzein, thymol blue, xylenol orange, 8-hydroxypyrene-1,3,6-trisulfonic acid, 5(6)-carboxyfluorescein, 5(6)-carboxynaphthofluorescein, carboxy SNAFL-1, carboxy SNAFL-2, carboxy SNARF-1, fluorescein diacetate, fluorescein-5-isothiocyanate, fluorexon, harmine, 4-methylumbelliferone, β-naphthol, umbelliferone. Suitable indicators of halide ions include lucigenin, *N*-dodecyl-6-methoxyquinolinium iodide, 6-methoxy-*N-*(3-sulfopropyl)quinolinium, *N*-(ethoxycarbonylmethyl)-6-methoxyquinolinium bromide, 6-methoxy-*N*-ethylquinolinium iodide, 6-methoxy-*N*-(8-octanoic acid) quinolinium tetraphenyl borate, 6-methoxy-*N*-(8-octanoic acid) quinolinium bromide, 6-methyl-*N*-(methyl) quinolinium bromide, 6-methyl-*N*-(methyl) quinolinium iodide, *N*-(sulphopropyl) acridinium, 3-(10-methylacridinium-9-yl) propionic acid tetrafluroboron, *N*-methyl-9-aminoacridinium, 10-methylacridinium-9-carboxamide, *N*-methylacridinium-9-methylcarboxylate, 1-methyl-2-(11-undecanoic acid)-9H-pyrido[3,4-b] indolium bromide, 1-methyl-2-(sulphonatopropyl)-9H-pyrido[3,4-b] indolium. The indicators used in preferred embodiment can be also derivatives of the above-mentioned indicators or conjugates of indicators with molecules such as proteins or polysaccharides. Also mixture of several indicators can be used to extend pH or halide ion sensitivity.

The indicator may be applied onto the carrier by any suitable technique. In particular, the carrier may be impregnated with the indicator, or the indicator may be applied onto the surface of the carrier or onto the surface of a layer carrying the enzyme.

In one preferred embodiment, the carrier is a piece of cellulose-based material such as paper or a plastic material with pH or halide ion indicator preferably in the form of a band or a stripe. The carrier can also be a piece of cellulose-based material such as paper or a plastic material with an incorporated pad impregnated with a pH or halide ion indicator, preferably in the form of a band or a strip. For instance, the carrier with an indicator may be a commercially available pH indicator stripe.

The haloalkane dehalogenases (EC 3.8.1.5) are enzymes showing activity in dehalogenation of halogenated aliphatic hydrocarbons. This invention includes wild-type haloalkane dehalogenases as well as their modified counterparts, e.g., genetically modified counterparts, which maintain dehalogenating activity. The modified enzymes may have at least 40 % identity, at least 50 % identity, at least 60 % identity, at least 70 % identity, at least 80 % identity, at least 90 % identity, or at least 95 % identity of amino acid sequence to wild-type haloalkane dehalogenases, provided they show the dehalogenating activity to halogenated aliphatic hydrocarbons. The minimum dehalogenating activity may be defined as specific activity of 1.1 nmol.s⁻¹.mg⁻¹, i.e., any activity equal to or higher than this threshold is considered to be dehalogenating activity in this invention. The changes in amino acid sequence can be achieved by mutations in various positions of the protein chain, and/or by extending or shortening the sequence.

In a preferred embodiment, at least one haloalkane dehalogenase selected from the family of enzymes EC 3.8.1.5 is used, e.g., haloalkane dehalogenase selected from the group comprising LinB from *Sphingobium japonicum* UT26, DhaA from *Rhodococcus rhodochrous* NCIMB 13064, DmbA from *Mycobacterium bovis* 5033/66, DmbB from *Mycobacterium bovis* 5033/66, DmbC from *Mycobacterium bovis* 5033/66, DbjA from *Bradyrhizobium japonicum* USDA110, DhlA from *Xanthobacter autotrophicus* GJ10, DatA from *Agrobacterium tumefaciens* C58, DbeA from *Bradyrhizobium elkanii* USDA94, DrbA from *Rhodopirellula baltica* SH1, DpcA from *Psychrobacter cryohalolentis* K5, DppA from *Plesiocystis pacifica* SIR-1, DhmA from *Mycobacterium avium* N85, DmlA from *Mesorhizobium loti* MAFF303099, DmtA (Rv2579) from *Mycobacterium tuberculosis* H37Rv, DmaA (DmxA) from *Marinobacter* sp. ELB17, DmmA (CurN) from metagenome of marine microbial consortium, HanR from *Rhodobacteraceae* bacterium UDC319, DadB from *Alcanivorax dieselolei* B-5, DspA from *Strongylocentrotus purpuratus.* This preferred embodiment also includes modified counterparts of the listed enzymes having at least 40 % identity, at least 50 % identity, at least 60 % identity, at least 70 % identity, at least 80 % identity, at least 90 % identity, or at least 95 % identity of amino acid sequence, and maintaining the dehalogenating activity.

The enzyme may be applied on the surface of said carrier or impregnated in said carrier or in a matrix or layer on top of said carrier, or immobilized on the carrier by any other suitable technique.

The immobilization may be achieved by means of a matrix for embedding the enzyme, by adsorption, covalent or affinity binding. The enzyme can be entrapped into organic matrices, e.g., dextran, starch, gelatin, chitin, agarose, kappa-carrageenan, cellulose, pectins, polyurethane, polyacrylamide, polyethylenimine, poly(propyl acrylic acid), poly(vinyl) alcohol, ethylcellulose, poly(2-hydroxyethyl methacrylate) or inorganic stable matrices, e.g., silica sol-gels prepared by hydrolytic polymerization of tetraalkoxysilanes. Also matrix composed of mixture of organic and inorganic monomers may be used, e.g., ORMOCER. The enzyme may be cross-linked after entrapment using cross-linking reagent such as glutaraldehyde, dextran polyaldehyde, 1-ethyl-3-(3-dimethylaminopropyl) carbodiimide, disuccinimidyl suberate. Enzyme may be also precipitated and cross-linked, thus forming cross-linked enzyme aggregates (Cao L., van Rantwijk F., Sheldon R.A. (2000): Cross-linked enzyme aggregates: a simple and effective method for the immobilization of penicillin acylase. Org. Lett. 2: 1361-1364), and subsequently entrapped into the matrix. The immobilization by adsorption can be achieved by non-covalent binding of enzyme to copolymer gels of acrylamide/maleic acid or itaconic acid, to ion-exchange resins such as polymers of poly(styrene-co-divinylbenzene) (Amberlite, Dowex, Diaion), derivatives of polyacrylamide (Duolite), derivatives of synthetic polymers (PMMAPEI) or derivatives of cross-linked polysaccharides (DEAE-cellulose, QAE-cellulose, SP-cellulose, DEAE-Sephadex, QAE-Sephadex, CM-Sephadex, DEAE-dextran, CH-Sepharose 4B (-NH(CH₂)+COOH), AH-Sepharose 4B (-NH-(CH₂)₆-NH₂), Q-Sepharose (-CH₂-N⁺(CH₃)₃), S-Sepharose (-CH₂-SO₃-), CM-Sepharose (OCH₂COOH). Further, the enzyme can be bound to the carrier possessing primary amine, carboxyl, sulfhydryl or carbonyl groups using a cross-linking agent, e.g., glutaraldehyde, dextran polyaldehyde, 1-ethyl-3-(3-dimethylaminopropyl) carbodiimide, *N*-(ε-maleimidocaproic acid)hydrazide, *N*-(α-maleimidoacetoxy)-succinimide ester, *N*-(β-maleimidopropyloxy)succinimide ester, *bis*(sulfosuccinimidyl)suberate, *bis*(sulfosuccinimidyl)glutarate, dimethyl pimelimidate·2HCl, dimethyl suberimidate·2HCl, disuccinimidyl glutarate, disuccinimidyl suberate, dithio-bis-maleimidoethane, *N*-ε-maleimidocaproic acid, *N*-(ε-maleimidocaproyloxy)succinimide ester, NHS-LC-diazirine, succinimidyl 6-(3'-[2-pyridyldithio]propionamido)hexanoate, *m*-maleimidobenzoyl-*N*-hydroxysuccinimide ester, *N-*hydroxysuccinimide, 2-pyridyldithiol-tetraoxatetradecane-*N*-hydroxysuccinimide, succinimidyl 3-(bromoacetamido)propionate, *N*-succinimidyl iodoacetate, NHS-PEGₓ-maleimide, succinimidyl-(4-psoralen-8-yloxy)butyrate, sulfosuccinimidyl(4-iodo-acetyl)aminobenzoate, *tris*-(succimimidyl aminotricetate). Also bioaffinity interactions between wild type or altered enzyme haloalkane dehalogenase may be used for immobilization on the carrier, e.g., interactions between histidine-tagged enzyme and immobilized metal ions including nickel, cobalt and copper, interaction between enzyme and respective antibody, use of enzymatically active fusion proteins interacting with immobilized substrate or inhibitor. The enzyme haloalkane dehalogenase used for preparation of detection stripes may be in the form of dissolved, crystalline, lyophilized or precipitated enzyme.

The present disclosure further provides a method for preparation of the device of the invention, which includes the step of immobilizing at least one haloalkane dehalogenase on a carrier and the step of applying an indicator of presence of halogenated aliphatic hydrocarbons onto the carrier. The steps can be carried out in any order. In one embodiment, the step of immobilizing the enzyme(s) can be performed by first applying a matrix onto the carrier, subsequently applying the haloalkane dehalogenase and optionally an additive, such as bovine serum albumin (BSA), onto the matrix layer, and subjecting the enzyme(s) to cross-linking. Immobilization of enzyme can be also performed by mixing enzyme(s) with a matrix followed by subsequent application onto the carrier and subjecting the enzyme to cross-linking. Preferably, the enzyme is co-immobilized with BSA, which increases activity retention due to providing functional groups for cross-linking of the enzyme with a low content of surface primary amines, carboxyls, sulfhydryls or carbonyls. The step of applying an indicator of presence of halogenated aliphatic hydrocarbons onto the carrier may be performed by impregnating the carrier with the indicator or applying the indicator directly onto the matrix layer containing the enzyme(s), or by any other suitable technique.

The present invention also provides a method of detecting at least one halogenated aliphatic hydrocarbon in a sample or environment, wherein the sample or environment is contacted with the device according to the invention pre-treated by hydration, and the chemical change of the indicator of presence of halogenated aliphatic hydrocarbons is evaluated. Hydration agents can include, e.g., buffers such as HEPES buffer, potassium phosphate buffer, sodium phosphate buffer, Tris-sulfate buffer, glycine buffer, CHES buffer, sodium bicarbonate buffer, mixtures of these buffers with organic water-miscible solvents, ionic liquids and/or deep eutectic solvents such as dioxane, acetone, acetonitrile, methanol, dimethyl sulfoxide, *tert*-butanol, dimethyl formamide, tetrahydrofuran, ethaline, glyceline, maline, oxaline, reline, 1,3-dimethylimidazolium methylsulfate [Mmim][MeSO₄], cocosalkyl pentaethoxy methyl ammonium methosulfate [CPMA][MS], 1-butyl-3-methylimidazolium tetrafluoroborate [Bzmim][BF₄], 1-butyl-3-methylimidazolium chloride [Bzmim]Cl, 1,3-dimethylimidazolium chloride [Dmim]Cl and 1-ethyl-3-methylimidazolium acetate [Emim][CH₃COO]. The water content in these mixtures can be within the range of 20 % to 100 % (v/v).

In a preferred embodiment, a control assay for pH active components of the sample or the environment or for halide ions present in the sample or the environment is performed simultaneously, using a detection device not containing the haloalkane dehalogenase, such as a common non-modified pH indicator stripe or a stripe carrying halide ion indicator.

The sample or environment to be analysed by the detection device may be in gaseous, liquid or solid phase such as gas, vapours, aerosol, surface water, groundwater, wastewater, sewage, industrial water discharge, landfill leachate, fertilizer and pesticide solutions, urban runoff of untreated waste water, blood, urine, soil, sludge, sediment, munition fragments, packing material, vegetation and tissue. The sample may be preprocessed prior to an analysis, e.g., filtration, centrifugation, preconcentration or extraction may be used.

The detection of halogenated aliphatic hydrocarbons can be performed in the temperature range of 10 to 60 °C with reaction optimum around 40 °C. The pH activity profile is broad, the detection can be thus carried out in pH interval from 4 to 10.

The colour change might be evaluated visually. Preferably, the colour change is evaluated by photographing the detection device by a digital image-processing device, such as a digital camera or a smartphone, tablet, scanner, after contacting said detection device with the sample or environment. Obtained image is subsequently split into the red, green and blue channel information, and obtained pseudocolor image is dividing the intensity data in one colour channel by the intensity data in another colour channel. Thus, semiquantitative information is obtained. Similarly, the fluorescence may be evaluated using a digital image-processing device equipped with suitable excitation source and by subsequent splitting such image into the red, green and blue channel information.

### Brief Description of Drawings

Figure 1. Calibration plot for gaseous bis(2-chloroethyl) ether (A) and sulfur mustard (B) determined using the test stripes based on pH indicator and haloalkane dehalogenase LinB, evaluated using the RGB readout.
Figure 2. Response of test stripes based on chloride-sensitive indicator and haloalkane dehalogenase LinB to bis(2-chloroethyl) ether.
Figure 3. The pH (A) and temperature (B) profile of immobilized LinB isolated from *Sphingobium japonicum* UT26. The enzyme is a part of detection device exposed to 0.34 mM 1,2-dibromoethane in 1 mM HEPES buffer.
Figure 4. Calibration plots of the detection device utilizing wild-type haloalkane dehalogenase (A) and a mutant of haloalkane dehalogenase (B) for 1,2,3-trichloropropane in 1 mM HEPES buffer (pH 9.0) at 22 °C. Concentrations of 1,2,3-trichloropropane were determined by gas chromatography.
Figure 5. Substrate specificity of selected haloalkane dehalogenases. Activities were measured with 30 different halogenated aliphatic hydrocarbons in 100 mM glycine buffer, pH 8.6 at 37 °C. The columns of specific activities higher than 80 nmol·s⁻¹·mg⁻¹ were trimmed for clarity (Koudelakova T., Bidmanova S., Dvorak P., Pavelka A., Chaloupkova R., Prokop Z., Damborsky J. (2012): Haloalkane dehalogenases: Biotechnological applications. Biotechnol. J. 8: 32-45).

### Examples of carrying out the invention

### Example 1

### Detection of industrial chemical bis(2-chloroethyl) ether and chemical warfare agent sulfur mustard in gaseous phase using the test stripes based on pH indicator and haloalkane dehalogenase LinB isolated from Sphingobium japonicum UT26.

The stripes were prepared by spreading the solution of HydroMed D4 hydrogel, 5 % (w/w) solution in ethanol/water (90/10, v/v), onto neutral and alkaline pH-sensitive panel of the pH stripes (50 µl per stripe) using a knife-coating device. The thickness of the layer was 0.125 mm. The hydrogel was allowed to dry for 1 h in air at 23 °C. The LinB enzyme (lyophilized in 50 mM phosphate buffer, pH 7.5, 50 mg) and powder of BSA (100 mg) were dissolved in 800 µl of water. Protein mixture (40 µl per stripe) was applied on the hydrogel layer stepwise (increments of 10 µL of immobilization mixture). Stripes were exposed to glutaraldehyde vapors for 30 min. Prepared test stripes were stored in a dark, cool and dry place before measurement.
Bis(2-chloroethyl) ether was diluted to required concentrations using methanol. The mixture (5 µl) was injected into an airtight vial and allowed to evaporate for 30 min at 23 °C. Gaseous concentrations of analytes were determined using gas chromatograph coupled with a mass spectrometer (Trace MS 2000, Finnigan, USA).
The gaseous sulfur mustard was generated by evaporating 0.5 ml of the sulfur mustard in a teflon vessel by increasing the temperature in a vapor generation system. Nitrogen gas was pumped through the vessel after system stabilization and pushed the mustard vapors into home-made holder of the stripes. The experiments were performed at ambient temperature and humidity. The concentration of the gaseous sulfur mustard was verified by drawing the vapors (1.3 1) through a bilayer tenax adsorption tube. Subsequently, thermal desorption was followed by analysis using a gas chromatograph coupled to a mass spectrometer (EM640 GC-MS, Bruker, Germany).
The test stripes for detection of bis(2-chloroethyl) ether and sulfur mustard were incubated in 10 ml of 100 mM glycine buffer, pH 8.6 and in 10 mL of 20 % (v/v) dioxane in mixture with 100 mM glycine buffer, pH 8.6 at 23 °C for 20 min, respectively. Hydrated stripes were exposed to the gaseous analyte and photographed after 10 min of enzymatic reaction at 23 °C. First, the response of stripes to analyte was evaluated visually by comparison with a reference scale. Further quantification was done using the red-green-blue readout (RGB) of a digital camera. Digital camera (Canon EOS 1100D, Canon, Japan) equipped with a standard 18-125 mm objective (F 3.8 - 5.6 DC OS) was fixed in a distance of a 30 cm above the stripes. Stripes were illuminated by a halogen lamp (50 W, 12 V). The camera operated in manual mode with parameters set as follow: ISO sensitivity, 100; shutter speed, 1/500 s; focal length, 125 mm; exposure, 0 EV; white balance, custom. Images were stored in RAW format. For data evaluation and processing, alkaline pH-sensitive panel of the stripe was used for calculation of mean grey value. The images were cropped and saved as a 16-bit color TIF file using the software Bibble 5 Pro (Bibble Labs, USA). This file was then split into the red, green and blue channel information via ImageJ software (National Institute of Mental Health, USA). The green channel did not contain any information and was discarded. Next, the intensity data of the red channel image were divided by the data of the blue channel image (red/blue signal, R/B).

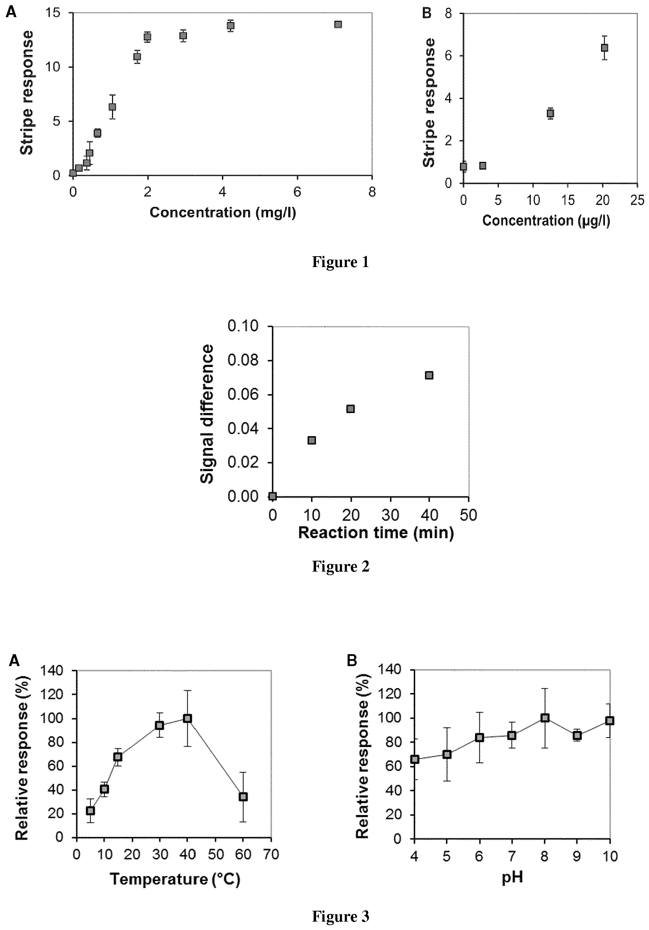

The response of test stripes to gaseous bis(2-chloroethyl) ether was expressed as the color change of the alkaline panel of the stripes from green to yellow with increasing concentrations of bis(2-chloroethyl) ether. High amounts of analyte also caused a color change from blue to green in the neutral panel of the stripes. Bis(2-chloroethyl) ether could be detected visually down to 2.0 mg·l⁻¹. A more accurate quantitative analysis was done using the RGB readout (see Figure 1A). The overall shape of calibration was sigmoidal with the saturation of the signal at concentrations of analyte above 2 mg/L. The calculated limit of detection was 70 µg·l⁻¹ for gaseous bis(2-chloroethyl) ether.

The test stripes were incubated in 20 % (v/v) dioxane prior detection of gaseous sulfur mustard. This increases the solubility of sulfur mustard in the stripe and suppresses its spontaneous hydrolysis. A color change of the test stripes was detected only upon exposure to saturated vapours of sulfur mustard. No color change was visible for stripes exposed to the concentrations from 0 to 20 µg·l⁻¹. Yet concentrations below 20 µg·l⁻¹ are well detectable using the RGB readout and exhibit a limit of detection of 3 µg·l⁻¹ (see Figure 1B).

### Example 2

### Detection of industrial chemicals bis(2-chloroethyl) ether and 1,2-dibromoethane in liquid phase using the test stripes based on pH indicator and haloalkane dehalogenase LinB isolated from Sphingobium japonicum UT26 and haloalkane dehalogenase DbjA isolated from Bradyrhizobium japonicum USDA110.

The stripes were prepared by spreading the solution of HydroMed D4 hydrogel, 5 % (w/w) solution in ethanol/water (90/10, v/v), onto neutral and alkaline pH-sensitive panel of the stripes (50 µl per stripe) using a knife-coating device. The thickness of the layer was 0.125 mm. The hydrogel was allowed to dry for 1 h in air at 23 °C. The LinB or DbjA enzyme (lyophilized in 50 mM phosphate buffer, pH 7.5, 50 mg) and powder of BSA (100 mg) were dissolved in 800 µL of water. Protein mixture (40 µl and 20 µl per stripe for LinB and DbjA, respectively) was applied on the hydrogel layer stepwise (increments of 10 µl of immobilization mixture). Stripes were exposed to glutaraldehyde vapors for 30 min. Prepared test stripes were stored in a dark, cool and dry place before measurement.
Performance of the test stripes was assessed by measurement of enzymatic activity and by visual evaluation of pH change on the stripes. Enzymatic activity of stripes hydrated in 100 mM glycine buffer (pH 8.6) for 0.5 h was assessed in 10 ml of 100 mM glycine buffer (pH 8.6) with 10 µl of bis(2-chloroethyl) ether or 1,2-dibromoethane at 21 °C by Iwasaki method (Iwasaki I., Utsumi S., Ozawa T. (1952): New colorimetric determination of chloride using mercuric thiocyanate and ferric ion. Bull. Chem. Soc. Jpn. 25: 226). The pH change accompanying enzymatic reaction was evaluated also visually by comparison of the enzymatic stripe with stripe without immobilized enzyme. The performance of test stripes was documented using digital camera (Canon PowerShot A700, Japan). The camera operated in automatic mode with macroimaging setting.

Prepared test stripes based on haloalkane dehalogenases LinB isolated from *Sphingobium japonicum* UT26 and DbjA isolated from *Bradyrhizobium japonicum* USDA110 were exposed to aqueous solutions containing industrial chemicals bis(2-chloroethyl) ether and 1,2-dibromoethane (see Table 2). The stripes based on haloalkane dehalogenases were active with both chemicals. However, the response of the stripes with immobilized DbjA enzyme is lower to bis(2-chloroethyl) ether compared to the stripes with immobilized LinB enzyme and the activity is detectable only using Iwasaki method. The test stripes based on DbjA enzyme are useful for determination of 1,2-dibromoethane, where significant activity and color change were observed.

**Table 2. Summary of the responses of test stripes based on pH indicator and haloalkane dehalogenases LinB and DbjA to industrial chemicals.**

| **Haloalkane dehalogenase** | **Analyte** | **Activity (µmol·s⁻¹)** | **pH** | |
|---|---|---|---|---|
| | | | 0 min | 10 min |
| LinB | Bis(2-chloroethyl) ether | 0.0027 ± 0.0003 | 8.5 | 7.0-7.5 |
| DbjA | Bis(2-chloroethyl) ether | 0.0011 ± 0.0003 | 8.5 | 8.0-8.5 |
| DbjA | 1,2-dibromoethane | 0.0076 ± 0.0014 | 8.5 | 7.0-7.5 |

### Example 3

### Detection of industrial chemical bis(2-chloroethyl) ether in liquid phase using test stripes based on chloride ion indicator and haloalkane dehalogenase LinB isolated from Sphingobium japonicum UT26.

Solution of D4 hydrogel (5 %, w/w) in ethanol/water (90/10, v/v) was spread onto pH-sensitive panel of pH stripes (50 µl per stripe) using a knife-coating device. The thickness of the layer was 0.125 mm. The hydrogel was allowed to dry for 1 h in air at 22 °C. Enzyme LinB (lyophilized in 50 mM phosphate buffer, pH 7.5, 20 mg) and BSA (40 mg) were dissolved in 320 µl distilled water. Protein mixture (20 µl per stripe) was applied on the hydrogel layer stepwise (increments of 10 µl of immobilization mixture). Stripes were exposed to glutaraldehyde vapours for 30 min. Fluorescent dye lucigenin (5 mg) and polyacrylonitrile nanoparticles with Ru(dpp)₃TMS₂ (30 mg) were added to 10 ml of 5 % (w/w) D4 hydrogel in ethanol/water (90/10, v/v). The mixture was vortexed and then ultrasonicated for 1 h. Mixture of fluorescent dyes (50 µl per stripe) was spread onto immobilized enzyme. The thickness of the layer prepared using the knife-coating device was 0.125 mm. Prepared dehalogenase-based stripes were incubated in 10 ml of 100 mM glycine buffer (pH 8.6) at 22 °C, after drying for 1 h in air at 22 °C. Hydrated stripes were exposed to 8.5 mM bis(2-chloroethyl) ether in 10 ml of 100 mM glycine buffer (pH 8.6) at 22 °C.
The stripe response to increasing concentration of chlorides produced by dehalogenase reaction was quantified using RGB readout. Digital camera (Canon EOS 550D, Canon, Japan) equipped with a standard 18-135 mm objective and an yellow glass filter (530 nm high-pass filter) was fixed on top of a black box. Camera operated in manual mode with parameters set as follow: aperture, 5.6; ISO sensitivity, 200; shutter speed, 1/50 s. Fluorescent images were obtained following photoexcitation with an array of 405 nm LEDs and stored in RAW format. The color temperature was set to 7200 K and exposure was set to 2.5 for all photographs using the software Bibble 5 Pro (Bibble Labs, USA). The images were saved as a 16-bit color TIF file and subsequently split into the red, green and blue channel information via ImageJ software (National Institute of Mental Health, USA). The blue channel did not contain any information and was discarded. Next, the intensity data of the red channel image were divided by the data of the green channel image (R/G signal) to give pseudocolor images. Signal difference was calculated as a difference between R/G signal obtained for particular and zero chloride concentration.

The test stripes based on chloride ion indicators and haloalkane dehalogenase LinB were used for detection of 8.5 mM bis(2-chloroethyl) ether in 100 mM glycine buffer (pH 8.6) at 22 °C (see Figure 2). The stripes enabled detection of this industrial chemical, the enzymatic reaction was accompanied by increase of signal difference as a function of time.

### Example 4

### Immobilization of haloalkane dehalogenase LinB isolated from Sphingobium japonicum UT26.

The enzyme haloalkane dehalogenase LinB was immobilized on the glass slides used as a carrier using previously described immobilization procedure (Bidmanova S., Damborsky J., Prokop Z. (2013): Immobilization of haloalkane dehalogenase LinB from Sphingobium japonicum UT26 for biotechnological applications. J. Biocatal. Biotransformation 2: 1-7). Enzymatic activity was determined for immobilized enzyme exposed to 10 ml of 100 mM glycine buffer (pH 8.6) for 24 h at 4 °C. Activity was measured in 10 ml of 100 mM glycine buffer (pH 8.6) at 37 °C, using the Iwasaki method (Iwasaki I., Utsumi S., Ozawa T. (1952): New colorimetric determination of chloride using mercuric thiocyanate and ferric ion. Bull. Chem. Soc. Japan. 25: 226). The substrate 1,2-dibromoethane was added to a final concentration of 8.7 mM. The reaction was started by addition of the enzyme to the reaction mixture, and terminated by mixing with 35 % (v/v) nitric acid. Increasing concentrations of bromide ions were measured with mercuric thiocyanate and ferric ammonium sulfate at 460 nm by spectrophotometer Sunrise.

Different methods and their combinations were used for immobilization of haloalkane dehalogenase LinB on the carrier (see Table 3). The immobilized enzyme was exposed to glycine buffer for 24 h to remove unbound molecules and the residual enzymatic activity was measured. The best results with the 47 %, 41 % and 33 % retention of initial enzymatic activity were obtained by using glutaraldehyde, 1-ethyl-3-(3-dimethylaminopropyl)carbodiimide with adipic acid dihydrazide and cross-linked enzyme aggregates methodology, respectively (Bidmanova S., Damborsky J., Prokop Z. (2013): Immobilization of haloalkane dehalogenase LinB from Sphingobium japonicum UT26 for biotechnological applications. J. Biocatal. Biotransformation 2: 1-7). Following entrapment of cross-linked enzyme aggregates into polyvinyl alcohol particles, with final 25 % retention of the initial enzymatic activity, secures high degree of protection for the enzyme against harsh conditions (Bidmanova S., Hrdlickova E., Jaros J., Ilkovics L., Hampl A., Damborsky J., Prokop Z. (2014): Microscopic monitoring provides information on structure and properties during biocatalyst immobilization. Biotechnol. J. 9: 1-9).

**Table 3. Effect of immobilization procedure on activities of haloalkane dehalogenase LinB. Activity was measured with 1,2-dibromoethane after 24 hours of exposure to 100 mM glycine buffer, pH 8.6. The standard errors were calculated from three independent measurements.**

| **Immobilization method** | **Immobilization reagent** | **Enzymatic activity (%)*** |
|---|---|---|
| Cross-linking | Glutaraldehyde | 46.5 ± 3.7 |
| Cross-linking on functionalized surface | Glutaraldehyde, (3-Aminopropyl)triethoxysilane | 23.7 ± 0.0 |
| Cross-linking | Dextran polyaldehyde | 18.7 ± 9.0 |
| Cross-linking on functionalized surface | Dextran polyaldehyde, (3-Aminopropyl)triethoxysilane | 11.3 ± 1.0 |
| Cross-linking | 1-ethyl-3-(3-dimethylaminopropyl)-carbodiimide | 25.3 ± 0.3 |
| Cross-linking | 1-ethyl-3-(3-dimethylaminopropyl)-carbodiimide, Adipic acid dihydrazide | 41.0 ± 2.1 |
| Cross-linking after precipitation | Dextran polyaldehyde | 33.0 ± 6.7 |
| Cross-linking after entrapment | Glutaraldehyde, ORMOCER | 12.7 ± 0.4 |
| Entrapment after cross-linking and precipitation | Dextran polyaldehyde, ORMOCER | 10.9 ± 0.8 |
| Entrapment after cross-linking and precipitation | Dextran polyaldehyde, Polyvinyl alcohol | 25.2 ± 1.3 |

| | | |
|---|---|---|
| * The activity of 100 % corresponds to the activity of soluble haloalkane dehalogenase LinB with 8.7 mM of standard substrate 1,2-dibromoethane in 100 mM glycine buffer (pH 8.6) at 37 °C. | | |

### Example 5

### Effect of temperature and pH on detection using haloalkane dehalogenase LinB isolated from Sphingobium japonicum UT26.

The effects of temperature and pH on detection were studied using haloalkane dehalogenase LinB as the model system. Mixture (5 µl) containing lyophilized haloalkane dehalogenase LinB (2 mg), fluorescence pH indicator 5(6)-carboxynaphthofluorescein conjugated with BSA (4 mg) and 32 µl of 25 % (v/v) glycerol was applied by the pipette on the surface of plastic support material into thin layer. Reference was prepared by application of mixture (5 µl) consisting of BSA (2 mg), fluorescence pH indicator 5(6)-carboxynaphthofluorescein conjugated with BSA (4 mg) and 32 µl of 25 % (v/v) glycerol on the surface of plastic support material. Both types of supports were exposed to 70 % (v/v) glutaraldehyde vapors for 30 min at 22 °C and then stored for 24 h in 50 mM phosphate buffer (pH 9.0) at 4 °C. The fluorescence signal from biosensor layer was recorded by using pocket-size fluorometer (Photon Systems Instruments, Czech Republic) with solid state LED containing a 590 nm band limiting filter as light source and PIN photodiode with a 670 nm band limiting filter as detector. The output signal was sent to computer and recorded using FluorPen 1.0 software (Photon Systems Instruments, Czech Republic). Effect of starting pH on activity of haloalkane dehalogenase was measured in pH range from 2.0 to 11.0 at 22 °C. Temperature dependence was investigated at fixed pH of HEPES buffer (9.0) in temperature range from 5 to 60 °C. All experiments were performed in triplicates with reference used as a control.

The detection of halogenated hydrocarbons is strongly dependent on temperature (see Figure 3A). The detection can be achieved using haloalkane dehalogenase LinB in the temperature range 10-60 °C with reaction optimum around 40 °C. Temperature of 5 °C is limiting due to the response of approximately 20 % and large scatter of data. The pH activity profile is also broad and allows detection in pH interval from 4-10 while maintaining reasonable activity (see Figure 3B).

### Example 6

### Detection of industrial chemical 1,2,3-trichloropropane in liquid phase using wild-type haloalkane dehalogenase LinB isolated from Sphingobium japonicum UT26 and engineered variant of haloalkane dehalogenase DhaA31 isolated from Rhodococcus rhodochrous NCIMB 13064.

Detection of 1,2,3-trichloropropane was tested with device based on wild-type haloalkane dehalogenase LinB from *Sphingobium japonicum* UT26 and the mutant of haloalkane dehalogenase DhaA from *Rhodococcus rhodochrous* NCIMB 13064. The mutant was constructed using a rational design combined with saturation mutagenesis and possessed five amino-acid substitutions: I135F, C176Y, V245F, L246I and Y273F (DhaA31, Pavlova M., Klvana M., Prokop Z., Chaloupkova R., Banas P., Otyepka M., Wade R.C., Nagata Y., Damborsky J. (2009): Redesigning dehalogenase access tunnels as a strategy for degrading an anthropogenic substrate. Nat. Chem. Biol. 5: 727-733). The plasmid pET21b was used for subcloning of genes of haloalkane dehalogenases LinB and DhaA31. The resulting constructs were transformed into competent cells of *E. coli* BL21 using the heat shock method and plated on LB agar plates with ampicillin (100 µg·ml⁻¹). Plates were incubated overnight at 37 °C. Single colonies were used to inoculate 10 ml of LB medium with the ampicillin (100 µg·ml⁻¹), and cells were grown at 37 °C. Overnight cultures were used to inoculate 1 1 of LB medium with the above mentioned antibiotic. Cells were cultivated at 37 °C with shaking until an optical density of 0.4-0.6 (λ = 600 nm), after which expression was induced with 0.5 mM isopropyl-β-D-thiogalactopyranoside. Cells were then cultivated overnight at 20 °C. Biomass was harvested by centrifugation.

Harvested cells containing haloalkane dehalogenases were washed and resuspended in purification buffer A (20 mM K₂HPO₄ and KH₂PO₄, 0.5 M NaCl, 10 mM imidazole, pH 7.5). DNase I was added in amount of 1 U/ml of cell suspension. Cells were disrupted by sonication using the ultrasonic processor Hielscher UP200S (Teltow, Germany) with 0.3 s pulses and 85 % amplitude. Cell lysate was centrifuged for 1 hour at 21,000 g at 4 °C, and the resulting cell free extract was decanted. The enzymes were purified using single-step metaloaffinity chromatography. Crude extract was applied to a 5 mL Ni-NTA Superflow column (Qiagen, Germany). The column was attached to a BioLogic Duo Flow (Bio-Rad, USA). The buffer system consisted of buffer A and buffer B (20 mM K₂HPO₄ and KH₂PO₄, 0.5 M NaCl, 500 mM imidazole, pH 7.5). Recombinant enzymes were eluted at 60 % of buffer B during a two-step gradient method: 0-10 % in 5 column volumes and 10-60 % of buffer B in 10 column volumes. Fractions containing enzyme were pooled and the protein was concentrated using a stirred ultrafiltration cell (Millipore, USA). The enzymes were dialyzed against 50 mM phosphate buffer (pH 7.5) and lyophilized using freeze dryer ALPHA 1-2 LD (Martin Christ, Germany).

Mixture (5 µl) containing lyophilized haloalkane dehalogenase LinB or DhaA31 (2 mg), fluorescence pH indicator 5(6)-carboxynaphthofluorescein conjugated with BSA (4 mg) and 32 µl of 25 % (v/v) glycerol was applied by the pipette on the surface of plastic support material into a thin layer. Reference was prepared by application of mixture (5 µl) consisting of BSA (2 mg), fluorescence pH indicator 5(6)-carboxynaphthofluorescein conjugated with BSA (4 mg) and 32 µl of 25 % (v/v) glycerol on the surface of plastic support material. Both supports were exposed to 70 % (v/v) glutaraldehyde vapors for 30 min at 22 °C and then stored for 24 h in 50 mM phosphate buffer (pH 9.0) at 4 °C. The fluorescence signal from biosensor layer was recorded by using pocket-size fluorometer (Photon Systems Instruments, Czech Republic) with solid state LED containing a 590 nm band limiting filter as light source and PIN photodiode with a 670 nm band limiting filter as detector. The output signal was sent to computer and recorded using FluorPen 1.0 software (Photon Systems Instruments, Czech Republic). All experiments were performed in triplicates with reference used as a control.

The measurements were performed in a stirred glass vial at 22 °C. The biosensor layer on plastic support was immersed into 5 ml of 1 mM HEPES buffer (pH 9.0). After recording the steady signal, 1,2,3-trichloropropane was injected into HEPES buffer (pH 9.0) and the signal was recorded for 30 min. Final response was calculated as a difference between initial and final fluorescence intensity. The used concentrations of 1,2,3-trichloropropane were determined by gas chromatograph GC Trace 2000 (Thermo Finnigan, USA) equipped with a capillary column DB-FFAP 30 m×0.25 mm×0.25 µm (Phenomenex, USA) and flame ionization detector. The calibration data were fitted using Origin 6.1 (OriginLab, USA). The detection limit was calculated as a signal three times the standard deviation of the noise related to the intercept of the linear function.

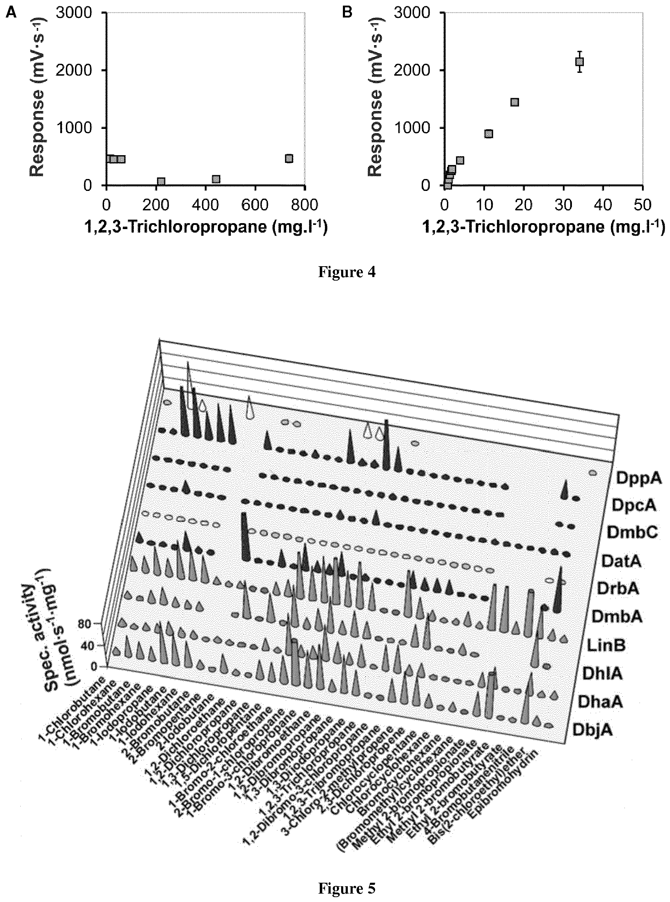

The device utilizing wild-type haloalkane dehalogenase LinB was used for detection of highly toxic recalcitrant pollutant 1,2,3-trichloropropane. The detection system was insensitive to this analyte (see Figure 4A) and thus, search for other haloalkane dehalogenase active with 1,2,3-trichloropropane was performed. Enzymatic activity was found for haloalkane dehalogenase DhaA, however, catalytic performance of the enzyme was still low (see Table 4). Thus, rational design was combined with saturation mutagenesis to obtain the haloalkane dehalogenase variant DhaA31 which displayed a 26-fold higher catalytic efficiency for 1,2,3-trichloropropane than the wild-type enzyme DhaA. Subsequent utilization of DhaA31 in the detection device provided calibration curve determined in the range from 0 to 40 mg·l⁻¹ of 1,2,3-trichloropropane. The exposure of the device to increasing concentrations of this analyte led to release of more protons from the enzymatic reaction, thus resulting in a higher response. A plateau was reached at a saturating concentration of the analyte. The calibration curve followed the Michaelis-Menten profile indicating an enzyme-catalyzed process with limit of detection of 1.4 mg·l⁻¹ and correlation coeficient of 0.99 (see Figure 4B).

**Table 4. Specific activities and steady-state kinetic parameters of wild-type haloalkane dehalogenases LinB, DhaA and the mutant DhaA31.**

| **Haloalkane dehalogenase** | **Specific activity (nmol·s⁻¹·mg⁻¹)** | ***K*ₘ (mM)** | ***k*_{cat} (s⁻¹)** | ***k*_{cat}*lK*ₘ (mM^{-1.}s⁻¹)** |
|---|---|---|---|---|
| LinB* | n.d. | n.d. | n.d. | n.d. |
| DhaA** | 1.8 | 0.98 | 0.04 | 0.04 |
| DhaA31** | 53.2 | 1.19 | 1.26 | 1.06 |

| | | | | |
|---|---|---|---|---|
| n.d. no activity detected under used experimental conditions * Koudelakova T., Bidmanova S., Dvorak P., Pavelka A., Chaloupkova R., Prokop Z., Damborsky J. (2012): Haloalkane dehalogenases: Biotechnological applications. Biotechnol. J. 8: 32-45. ** Pavlova M., Klvana M., Prokop Z., Chaloupkova R., Banas P., Otyepka M., Wade R.C., Nagata Y., Damborsky J. (2009): Redesigning dehalogenase access tunnels as a strategy for degrading an anthropogenic substrate. Nat. Chem. Biol. 5: 727-733. | | | | |

### Example 7

### Substrate specificities of selected haloalkane dehalogenases.

Substrate specificities of various wild-type haloalkane dehalogenases were tested with 30 different halogenated aliphatic hydrocarbons under standardized conditions. Genes of haloalkane dehalogenases were cloned into a plasmid. The resulting constructs were transformed into competent cells of *E. coli* using the heat shock method and plated on LB agar plates with antibiotic. Overnight cultures were used to inoculate cultivation medium and cells were cultivated with shaking until an optical density of 0.4-0.6 (λ = 600 nm), after which expression was induced. After further cultivation, biomass was harvested by centrifugation. Harvested cells containing haloalkane dehalogenases were washed and resuspended in purification buffer A (20 mM K₂HPO₄ and KH₂PO₄, 0.5 M NaCl, 10 mM imidazole, pH 7.5). DNase I was added into the cell suspension. Cells were disrupted by sonication using the ultrasonic processor Hielscher UP200S (Teltow, Germany) with 0.3 s pulses and 85 % amplitude. Cell lysate was centrifuged for 1 hour at 21,000 g at 4 °C, and the resulting cell-free extract was decanted.
The enzymes were purified using single-step metaloaffinity chromatography. Crude extract was applied to a 5 ml Ni-NTA Superflow column (Qiagen, Germany). The column was attached to a BioLogic Duo Flow (Bio-Rad, USA). The buffer system consisted of buffer A and buffer B (20 mM K₂HPO₄ and KH₂PO₄, 0.5 M NaCl, 500 mM imidazole, pH 7.5). Recombinant enzymes were eluted at 60 % of buffer B during a two-step gradient method: 0-10 % in 5 column volumes and 10-60 % of buffer B in 10 column volumes. Fractions containing enzyme were pooled and the protein was concentrated using a stirred ultrafiltration cell (Millipore, USA). The enzymes were dialyzed against 50 mM phosphate buffer (pH 7.5).
The enzymatic activity was determined in 10 ml of 100 mM glycine buffer (pH 8.6) at 37 °C using the Iwasaki method (Iwasaki I., Utsumi S., Ozawa T. (1952): New colorimetric determination of chloride using mercuric thiocyanate and ferric ion. Bull. Chem. Soc. Japan. 25: 226). The halogenated substrate (10 µl) was added into the glycine buffer. After dissolution, the reaction was started by addition of the enzyme to the reaction mixture, and terminated by mixing with 35 % (v/v) nitric acid. Increasing concentrations of reaction products (halide ions) were measured with mercuric thiocyanate and ferric ammonium sulfate at 460 nm by spectrophotometer Sunrise (Tecan, Switzerland). The amount of reaction products was determined from a calibration curve, prepared using sodium chloride, sodium bromide and potassium iodide as a standard solution.

Enzymatic activity was determined for haloalkane dehalogenases comprising DbjA from *Bradyrhizobium japonicum* USDA110, DhaA from *Rhodococcus rhodochrous* NCIMB 13064, DhlA from *Xanthobacter autotrophicus* GJ10, LinB from *Sphingobium japonicum* UT26, DmbA from *Mycobacterium bovis* 5033/66, DrbA from *Rhodopirellula baltica* SH1, DatA from *Agrobacterium tumefaciens* C58, DmbC from *Mycobacterium bovis* 5033/66, DpcA from *Psychrobacter cryohalolentis* K5G-I and DppA from *Plesiocystis pacifica* SIR-1. The study demonstrated broad substrate specificity of the selected haloalkane dehalogenases (see Figure 5). Tested enzymes were able to cleave carbon-halogen bond in more than thirty chlorinated, brominated and iodinated aliphatic hydrocarbons containing a monohalogenated *sp³* hybridized carbon as substrate. Some of the substrates could be classified as the "universal" substrates of analyzed haloalkane dehalogenases, i.e., 1-bromobutane, 1-iodopropane, 1-iodobutane, 1,2-dibromoethane and 4-bromobutanenitrile, while other belong to the "poor" substrates, i.e., 1,2-dichloropropane, 1,2,3-trichloropropane, chlorocyclohexane and (bromomethyl)cyclohexane (Koudelakova T., Bidmanova S., Dvorak P., Pavelka A., Chaloupkova R., Prokop Z., Damborsky J. (2012): Haloalkane dehalogenases: Biotechnological applications. Biotechnol. J. 8: 32-45). Knowledge of substrate specificity enables selection of proper enzyme for preparation of test stripes useful for detection of specific halogenated chemicals used as pesticides, solvents, chemical intermediates, cleaning and degreasing agents, gasoline additives, chemical warfare agents, etc. The selection of individual haloalkane dehalogenase based on substrate specificity was demonstrated in Example 1. Herein, haloalkane dehalogenase LinB isolated from *Sphingobium japonicum* UT26 was immobilized on the carrier to prepare test stripes detecting warfare agent sulfur mustard owing to recognized activity of LinB with this substrate (Prokop Z., Damborsky J., Opluštil F., Jesenská A., Nagata Y. (2005): Method of detoxification of yperite by using haloalkane dehalogenases. Masaryk University, Brno, Czech Republic, Patent WO 2006/128390 A1).

## Claims

1. A device for detection of halogenated aliphatic hydrocarbons **characterized in that** it contains a paper carrier with at least one indicator selected from a pH indicator with a pH-indicating change within the pH range of 4.0 to 10.0, and an indicator of halide ions, and at least one haloalkane dehalogenase (EC 3.8.1.5) immobilized on the surface of said carrier.

2. The device according to claim 1, wherein the haloalkane dehalogenase (EC 3.8.1.5) is selected from the group comprising LinB from *Sphingobium japonicum* UT26, DhaA from *Rhodococcus rhodochrous* NCIMB 13064, DmbA from *Mycobacterium bovis* 5033/66, DmbB from *Mycobacterium bovis* 5033/66, DmbC from *Mycobacterium bovis* 5033/66, DbjA from *Bradyrhizobium japonicum* USDA110, DhlA from *Xanthobacter autotrophicus* GJ10, DatA from *Agrobacterium tumefaciens* C58, DbeA from *Bradyrhizobium elkanii* USDA94, DrbA from *Rhodopirellula baltica* SH1, DpcA from *Psychrobacter cryohalolentis* K5, DppA from *Plesiocystis pacifica* SIR-1, DhmA from *Mycobacterium avium* N85, DmlA from *Mesorhizobium loti* MAFF303099, DmtA (Rv2579) from *Mycobacterium tuberculosis* H37Rv, DmaA (DmxA) from *Marinobacter* sp. ELB17, DmmA (CurN) from metagenome of marine microbial consortium, HanR from *Rhodobacteraceae* bacterium UDC319, DadB from *Alcanivorax dieselolei* B-5, DspA from *Strongylocentrotus purpuratus* and modified counterparts of these enzymes having at least 40 % identity, at least 50 % identity, at least 60 % identity, at least 70 % identity, at least 80 % identity, at least 90 % identity, or at least 95 % identity of the amino acid sequence, provided that it has dehalogenating activity.

3. The device according to any one of the preceding claims, further containing a matrix for embedding the enzyme, the enzyme being preferably cross-linked, entrapped or adsorbed and optionally containing at least one additive such as bovine serum albumine.

4. A method of detecting at least one halogenated aliphatic hydrocarbon in a sample or environment, **characterized in that** the device according to any one of claims 1 to 3 is pre-treated by hydration, the sample or environment is contacted with said device according to any one of claims 1 to 3 pre-treated by hydration, and the chemical change of the indicator of presence of halogenated aliphatic hydrocarbons is evaluated.

5. The method according to the claim 4, wherein the halogenated aliphatic hydrocarbon is selected from C1-C18 alkanes, C2-C18 alkenes, C3-C18 cycloalkanes, wherein the alkane, alkene or cycloalkane is substituted by at least one halogen (selected from F, Cl, Br, I) and optionally further substituted by one or more substituents F, Cl, Br, I, OH, COOH, CN, COO(C1-C4 alkyl), C(O)N(H, C1-C6 alkyl, C6-C10 aryl), N(H, C1-C6 alkyl, C6-C10 aryl)-C(O)-(C1-C4 alkyl), O(C1-C6 alkyl, C2-C6 alkenyl, C1-C6 haloalkyl, C2-C6 haloalkenyl), S(C1-C6 alkyl, C2-C6 alkenyl, C1-C6 haloalkyl, C2-C6 haloalkenyl).

6. The method according to any one of the claims 4 to 5, wherein the chemical change is an absorbance or fluorescence change which is evaluated by photographing the detection device by a digital image-processing device, after contacting said detection device with the sample or environment, and by subsequent splitting such image into the red, green and blue channel information, and obtaining a pseudocolor image by dividing the intensity data in one colour channel by the intensity data in another colour channel.

## Patentansprüche

1. Vorrichtung zum Nachweis von halogenierten aliphatischen Kohlenwasserstoffen, **dadurch gekennzeichnet, dass** sie einen Papierträger mit mindestens einem Indikator enthält, der aus einem pH-Indikator mit einer pH-Indikationsänderung innerhalb des pH-Bereichs von 4,0 bis 10,0 und einem Indikator für Halogenidionen ausgewählt ist; und mindestens eine Halogenalkan-Dehalogenase (EC 3.8.1.5), die auf der Oberfläche des Trägers immobilisiert ist.

2. Vorrichtung nach Anspruch 1, wobei die Halogenalkandehalogenase (EC 3.8.1.5) aus der Gruppe ausgewählt ist, die LinB aus *Sphingobium japonicum* UT26, DhaA aus *Rhodococcus rhodochrous* NCIMB 13064, DmbA aus *Mycobacterium bovis* 5033/66, DmbB aus *Mycobacterium bovis* 5033/66, DmbC aus *Mycobacterium bovis* 5033/66, DbjA aus *Bradyrhizobium japonicum* USDA110, DhlA aus *Xanthobacter autotrophicus GJ10*, DatA aus *Agrobacterium tumefaciens* C58, DbeA aus *Bradyrhizobium elkanii* USDA94, DrbA aus *Rhodopirellula baltica* SH1, DpcA aus *Psychrobacter cryohalolentis* K5, DppA aus *Plesiocystis pacifica* SIR-1, DhmA aus *Mycobacterium avium* N85, DmlA aus *Mesorhizobium loti* MAFF303099, DmtA (Rv2579) aus *Mycobacterium tuberculosis* H37Rv, DmaA (DmxA) aus *Marinobacter* sp. ELB17, DmmA (CurN) aus einem Metagenom eines marinen mikrobiellen Konsortiums, HanR aus *Rhodobacteraceae-Bakterium* UDC319, DadB aus *Alcanivorax dieselolei* B-5, DspA aus *Strongylocentrotus purpuratus* und modifizierten Gegenstücken dieser Enzyme mit mindestens 40% Identität, mindestens 50% Identität, mindestens 60% Identität, mindestens 70% Identität, mindestens 80% Identität, mindestens 90% Identität oder mindestens 95% Identität der Aminosäuresequenz, vorausgesetzt, dass sie dehalogenierende Aktivität aufweisen.

3. Vorrichtung nach einem der vorhergehenden Ansprüche, ferner enthaltend eine Matrix zum Einbetten des Enzyms, wobei das Enzym vorzugsweise vernetzt, eingeschlossen oder adsorbiert ist, und gegebenenfalls mindestens einen Zusatzstoff wie Rinderserumalbumin enthält.

4. Verfahren zum Nachweisen mindestens eines halogenierten aliphatischen Kohlenwasserstoffs in einer Probe oder Umgebung, **dadurch gekennzeichnet, dass** die Vorrichtung nach einem der Ansprüche 1 bis 3 durch Hydratation vorbehandelt wird, die Probe oder Umgebung mit der durch Hydratation vorbehandelter Vorrichtung nach einem der Ansprüche 1 bis 3 in Kontakt gebracht wird, und die chemische Änderung des Indikators der Anwesenheit von halogenierten aliphatischen Kohlenwasserstoffen wird bewertet.

5. Verfahren nach Anspruch 4, wobei der halogenierte aliphatische Kohlenwasserstoff ausgewählt ist aus C1-C18-Alkanen, C2-C18-Alkenen, C3-C18-Cycloalkanen, wobei das Alkan, Alken oder Cycloalkan durch mindestens ein Halogen (ausgewählt aus F, Cl, Br, I) und gegebenenfalls weiter substituiert durch einen oder mehrere Substituenten F, Cl, Br, I, OH, COOH, CN, COO(C1-C4-Alkyl), C(O)N(H, C1-C6-Alkyl, C6-C10-Aryl), N(H, C1-C6-Alkyl, C6-C10-Aryl)-C(O)-(C1-C4-alkyl), O(C1-C6-Alkyl, C2-C6-Alkenyl, C1-C6-Halogenalkyl, C2-C6-Halogenalkenyl), S(C1-C6-Alkyl, C2-C6-Alkenyl, C1-C6-Halogenalkyl, C2-C6-Halogenalkenyl).

6. Verfahren nach einem der Ansprüche 4 bis 5, wobei die chemische Änderung eine Absorptions- oder Fluoreszenzänderung ist, die durch Fotografieren der Nachweisvorrichtung durch eine digitale Bildverarbeitungsvorrichtung nach dem Kontaktieren der Nachweisvorrichtung mit der Probe oder Umgebung bewertet wird, und durch nachfolgendes Aufteilen eines solchen Bildes in die roten, grünen und blauen Kanalinformationen und Erhalten eines Pseudofarbbildes durch Teilen der Intensitätsdaten in einem Farbkanal durch die Intensitätsdaten in einem anderen Farbkanal.

## Revendications

1. Dispositif de détection d'hydrocarbures aliphatiques halogénés **caractérisé en ce qu'**il contient un support en papier avec au moins un indicateur choisi parmi un indicateur de pH avec un changement indicateur de pH dans la gamme de pH de 4,0 à 10,0 et un indicateur d'ions halogénures; et au moins une haloalcane déshalogénase (EC 3.8.1.5) immobilisée à la surface dudit support.

2. Dispositif selon la revendication 1, dans lequel l'halogénoalcane déshalogénase (EC 3.8.1.5) est choisie dans le groupe comprenant LinB de *Sphingobium japonicum* UT26, DhaA de *Rhodococcus rhodochrous* NCIMB 13064, DmbA de *Mycobacterium bovis* 5033/66, DmbB de *Mycobacterium bovis* 5033/66, DmbC de *Mycobacterium bovis* 5033/66, DbjA de *Bradyrhizobium japonicum* USDA110, DhlA de *Xanthobacter autotrophicus GJ10*, DatA de *Agrobacterium tumefaciens* C58, DbeA de *Bradyrhizobium elkanii* USDA94, DrbA de *Rhodopirellula baltica* SH1, DpcA de *Psychrobacter cryohalolentis* K5, DppA de *Plesiocystis pacifica* SIR-1, DhmA de *Mycobacterium avium* N85, DmlA de *Mesorhizobium loti* MAFF303099, DmtA (Rv2579) de *Mycobacterium tuberculosis* H37Rv, DmaA (DmxA) de *Marinobacter* sp. ELB17, DmmA (CurN) du métagénome du consortium microbien marin, HanR de *Rhodobacteraceae* bacterium UDC319, DadB de *Alcanivorax dieselolei* B-5, DspA de *Strongylocentrotus purpuratus* et homologues modifiés de ces enzymes ayant identité d'au moins 40 %, identité d'au moins 50 %, identité d'au moins 60 %, identité d'au moins 70 %, identité d'au moins 80 %, identité d'au moins 90 %, ou identité d'au moins 95 % de la séquence d'acides aminés, à condition qu'elle ait une activité déshalogénante.

3. Dispositif selon l'une quelconque des revendications précédentes, contenant en outre une matrice pour incorporer l'enzyme, l'enzyme étant de préférence réticulée, incorporée ou adsorbée, et contenant éventuellement au moins un additif tel que l'albumine de la sérum bovine.

4. Procédé de détection d'au moins un hydrocarbure aliphatique halogéné dans un échantillon ou un environnement, **caractérisé en ce que** le dispositif selon l'une quelconque des revendications 1 à 3 est prétraité par hydratation, l'échantillon ou l'environnement est mis en contact avec ledit dispositif selon l'une quelconque des revendications 1 à 3 prétraité par hydratation, et le changement chimique de l'indicateur de présence d'hydrocarbures aliphatiques halogénés est évalué.

5. Procédé selon la revendication 4, dans lequel l'hydrocarbure aliphatique halogéné est choisi parmi les alcanes en C1 à C18, les alcènes en C2 à C18, les cycloalcanes en C3 à C18, dans lesquels l'alcane, l'alcène ou le cycloalcane est substitué par au moins un halogène (choisi parmi F, Cl, Br, I) et éventuellement substitué de plus par un ou plusieurs substituants F, Cl, Br, I, OH, COOH, CN, COO (alkyle en C1 à C4), C(O)N(H, alkyle en C1 à C6, aryle en C6 à C10), N(H, alkyle en C1 à C6, aryle en C6 à C10)-C(O)-(alkyle en C1 à C4), O(alkyle en C1 à C6, alcényle en C2 à C6, halogénalkyle en C1 à C6, halogénoalcényle en C2 à C6), S(alkyle en C1 à C6, alcényle en C2 à C6, halogénoalkyle en C1 à C6, halogénoalcényle en C2 à C6).

6. Procédé selon l'une quelconque des revendications 4 à 5, dans lequel le changement chimique est un changement d'absorbance ou de fluorescence qui est évalué en photographiant le dispositif de détection par un dispositif de traitement d'image numérique, après avoir mis en contact ledit dispositif de détection avec l'échantillon ou l'environnement, et en fractionnant ensuite cette image en informations de canaux rouge, vert et bleu, et en obtenant une image pseudocolor en divisant les données d'intensité dans un canal de couleur par les données d'intensité dans un autre canal de couleur.
